(19) Europäisches Patentamt
European Patent Office
Office européen des brevets

(11) **EP 1 242 024 B1**

(12) **EUROPEAN PATENT SPECIFICATION**

(45) Date of publication and mention
of the grant of the patent:
**05.09.2007 Bulletin 2007/36**

(51) Int Cl.:
***A61F 13/15*** (2006.01)

(21) Application number: **99964043.6**

(22) Date of filing: **02.12.1999**

(86) International application number:
**PCT/US1999/028514**

(87) International publication number:
**WO 2001/039709 (07.06.2001 Gazette 2001/23)**

(54) **DISPOSABLE GARMENT USING NONWOVEN LAYER FORMED FROM HIGHLY ORIENTED COMPONENT FIBERS**

WEGWERFKLEIDUNGSSTÜCK MIT EINEM VLIESSTOFF AUS RICHTUNGSGEBUNDENEN FASERN

VETEMENT JETABLE A COUCHE NON TISSEE CONSTITUEE DE FIBRES COMPOSEES FORTEMENT ORIENTEES

(84) Designated Contracting States:
**AT BE CH CY DE DK ES FI FR GB GR IE IT LI LU MC NL PT SE**

(43) Date of publication of application:
**25.09.2002 Bulletin 2002/39**

(73) Proprietor: **THE PROCTER & GAMBLE COMPANY Cincinnati, Ohio 45202 (US)**

(72) Inventors:
• **Ohnishi, Kazuyuki Takaishi Osaka 592-0003 (JP)**
• **Ui, Yoko Kawasaki-shi, Kanagawa 211-0016 (JP)**

(74) Representative: **Kremer, Véronique Marie Joséphine et al Procter & Gamble Service GmbH Sulzbacher Strasse 40-50 65824 Schwalbach am Taunus (DE)**

(56) References cited:
**EP-A- 0 858 791        US-A- 4 741 941
US-A- 5 782 819**

**Description**

<u>FIELD</u>

The present invention relates to disposable garments. Examples of such disposable garments include disposable underwear, disposable diapers including pull-on diapers and training pants, and disposable panties for menstrual use. More specifically, the present invention relates to disposable garments using a nonwoven layer formed from highly oriented component fibers.

<u>BACKGROUND</u>

Infants and other incontinent individuals wear disposable garments such as diapers to receive and contain urine and other body exudates. One type of the disposable garments, which is often called as "tape type", has a fastener system to hold the disposable garment at the wearer's waist area. As the fastener system, either an adhesive tape system or a mechanical fastener system is often used. Recently, elastically stretchable ear panels tend to be preferably used in this type of disposable garment, because they can provide a better fit to the wearer's waist area by jointly working with the fastener system. U.S. Patent 5,782,819 discloses a disposable diaper having front and back waistband sections, an intermediate section interconnecting the front and back waistband section. At least a first fit panel is connected to an inside surface of at least one of the waistband sections and the fit panel has a direction-dependent coefficient of friction value along a basis line of the fit panel. The disposable diaper can optionally comprise side panel members. The side panel members may be integrally formed with a selected diaper component or may, alternatively, be provided by one or more separate members. Another type of disposable garments, which is often called as "pant type" or "pull-on", has fixed sides and has become popular for use on children able to walk and often who are toilet training. This type of pull-on garments has ear panels the edges of which are seamed together to form two leg openings and a waist opening. These pull-on garments need to fit snugly about the waist and legs of the wearer without drooping, sagging or sliding down from position on the torso to contain body exudates. Examples of these pull-on garments are disclosed, for example, in U.S. Patent No. 5,171,239 to Igaue et al., U.S. Patent No. 4,610,681 to Strohbeen et al., WO 93/17648 published on September 16, 1993, U.S. Patent No. 4,940,464 to Van Gompel et al., U.S. Patent No. 5,246,433 to Hasse et al., and U.S. Patent No. 5,569,234 to Buell et al.

Good performance characteristics of such stretchable ear panels are important for these types of disposable garments. More specifically, the stretch properties including the stretch forces, recovery forces, retention forces, and available stretch of the ear panels are important considerations in the performance of the fitness for pull-on garments. The stretch properties provide the applicator and the wearer with the overall perceived "stretchiness" during use. They also effect the ability of the applicator to achieve a suitable degree of application stretch.

To provide good performance characteristics in stretchable ear panels of disposable garments, ear panels which include an elastic material having suitable properties have been studied and applied to disposable garments. It is generally expected that disposable garments provide good fit to the body and/or skin of the user by using suitable elastic materials in ear panels during the entire use period of products. Typical examples of such ear panels that have been previously used include an elastic material (e.g., elastic strings) joined to a non(or less)-elastic material such as nonwoven fabrics or plastic films. These non(or less)-elastic materials tend to affect expected elastic properties of the ear panels. For example, those materials tend to decrease elastic "stretchiness" of the stretchable ear panels during use.

Recent disposable garments tend to have an ear panel which includes a part of a barrier cuff material and/or an outer cover material. Barrier cuffs are often used in recent disposable garments and include (a) a channel portion having a proximal edge joined to a topsheet and a the distal edge which is away from the topsheet, and (b) a flap portion which extends laterally outward from the proximal edge into a part of the ear panel. Such barrier cuffs are disclosed in, for example, U.S. Patent 4,795,454 entitled "Absorbent Article Having Leakage-Resistant Dual Cuffs" issued to Dragoo on January 3, 1989. Outer covers are also often used in recent disposable garments and include a soft and cloth-like nonwoven material which is joined to the garment-facing surface of a liquid impervious film of backsheet. Such a nonwoven outer cover material tends to extend laterally outward into a part of the ear panel.

A "zero strain" stretch laminate is also known in the art and is used in ear panels of disposable garments. Example of such ear panels are disclosed in U.S. Patent No. 5,246,433 issued to Hasse et al. on September 21, 1993. In a manufacturing process for such "zero strain" stretch laminate, the elastomeric material is operatively joined to at least one component material in a substantially untensioned (zero strain) condition. At least a portion of the resultant composite stretch laminate is then subjected to mechanical stretching sufficient to permanently elongate the non-elastic components. The composite stretch laminate is then allowed to return to its substantially untensioned condition. Thus, the elastic laminate is formed into a "zero strain" stretch laminate. The resulting stretch laminate is thereby rendered elastically stretchable, at least up to the point of initial stretching, in the direction of initial stretching.

As is noted in the above, the manufacturing process of such "zero strain" stretch laminate requires the step of subjecting the non-elastic composite stretch laminate to mechanical stretching sufficient to permanently elongate the non-elastic components. When ear panels of disposable garments have a part of a barrier cuff material and/or an outer cover material, the existence of this step gives limitations to such materials. For example, the barrier cuff and/or outer cover material(s) are required to have a lower stress when being stretched, otherwise those materials tend to affect the stretchability of the ear panels and/or the applicability of the garment to wearer. Specifically, if the barrier cuff and/or outer cover material(s) do not have a lower stress when being stretched, those tend to decrease the stretchability of the ear panels and/or the applicability of the garment to wearer.

Based on the foregoing, there is a need for disposable garments which include a barrier cuff material and/or an outer cover material that has a lower stress when being stretched. There is also another need for disposable garments which include a barrier cuff material and/or an outer cover material that do not require the mechanical stretching process (i.e., the "zero strain" stretch laminate formation process) in their manufacturing process.

SUMMARY

The present invention is directed to a disposable garment according to claim 1. The chassis includes a topsheet, a backsheet associated with the topsheet, and an absorbent core disposed between the topsheet and the backsheet. The disposable garment further comprises an outer cover disposed on the garment-facing surface of the backsheet and including a first fiber oriented nonwoven layer having a Fiber Orientation Ratio within about ±20 degrees from a primary fiber direction of at least about 65%.

The present invention is also directed to a disposable garment according to claim 2. The chassis includes a topsheet, a backsheet associated with the topsheet, and an absorbent core disposed between the topsheet and the backsheet. The disposable garment further comprises at least one pair of stretchable ear panels extending laterally outward from the chassis in the front or back region; and a barrier cuff includes (a) a channel portion having a proximal edge joined to the topsheet and a the distal edge which is away from the topsheet, and (b) a flap portion which extends laterally outward from the proximal edge into at least a part of the ear panel. At least the flap portion includes a second fiber oriented nonwoven layer having a Fiber Orientation Ratio within about ±20 degrees from a primary fiber direction of at least about 65%.

The present invention is further directed to a composite laminate according to claim 10. The water resistant layer has a water resistance of at least about 50 mmH$_2$O. The third fiber oriented nonwoven layer has a Fiber Orientation Ratio within about ±20 degrees from a primary fiber direction of at least about 65%.

The foregoing answers the need for disposable garments which include a barrier cuff material and/or an outer cover material that has a lower stress when being stretched and/or that does not require the mechanical stretching process (i.e., the "zero strain" stretch laminate formation process) in their manufacturing process.

These and other features, aspects, and advantages of the present invention will become evident to those skilled in the art from reading of the present disclosure.

BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:

Fig. 1 is a perspective view of one preferred embodiment of the disposable pull-on diaper of the present invention in a typical in use configuration;
Fig. 2 is a simplified plan view of the embodiment shown in Fig. 1 in its flat uncontracted condition showing the body-facing side of the garment;
Fig. 3 is a cross-sectional view of a preferred embodiment taken along the section line 3-3 of Fig. 2;
Fig. 4 is a cross-sectional view of an elastic laminate of a preferred embodiment; and
Fig. 5 is a fragmentary enlarged perspective illustration of one preferred example of the elastomeric material layer.

DETAILED DESCRIPTION

All cited references are incorporated herein by reference in their entireties. Citation of any reference is not an admission regarding any determination as to its availability as prior art to the claimed invention.

Herein, "comprise" and "include" mean that other element(s) and step(s) which do not affect the end result can be added. These terms encompass the terms "consisting of" and "consisting essentially of".

Herein, "pull-on garment" refers to articles of wear which have a defined waist opening and a pair of leg openings and which are pulled onto the body of the wearer by inserting the legs into the leg openings and pulling the article up over the waist.

Herein, "disposable" describes garments which are not intended to be laundered or otherwise restored or reused as a garment (i.e., they are intended to be discarded after a single use and, preferably, to be recycled, composted or otherwise disposed of in an environmentally compatible manner).

Herein, "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties, and the like.

Herein, "nonwoven" may include any material which has been formed without the use of textile weaving processes which produce a structure of individual fibers which are interwoven in an identifiable manner. Methods of making suitable nonwovens includes a carded nonwoven process, a spunbonded nonwoven process, or the like.

Herein, "panel" denotes an area or element of the pull-on garment. While a panel is typically a distinct area or element, a panel may coincide (functionally correspond) somewhat with an adjacent panel.

Herein, "layer" does not necessarily limit the element to a single strata of material in that a layer may actually comprise laminates or combinations of sheets or webs of the requisite type of materials.

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "uncontracted state" is used to describe states of pull-on garments in its unseamed (i.e., seams are removed), flat and relaxed condition wherein all elastic materials used are removed therefrom.

The present invention can be applied to a variety of disposable garments. Such disposable garments include disposable underwear, disposable diapers including pull-on diapers and training pants, and disposable panties for menstrual use.

In one aspect of the present invention, the disposable garment includes an outer cover disposed on the garment-facing surface of a backsheet and include a fiber oriented nonwoven layer having a Fiber Orientation Ratio within about ±20 degrees from a primary fiber direction of at least about 65%.

In another aspect of the present invention, the disposable garment includes a barrier cuff including a channel portion and a flap portion. At least the flap portion includes a fiber oriented nonwoven layer having a Fiber Orientation Ratio within about ±20 degrees from a primary fiber direction of at least about 65%.

Since the nonwoven layer of the present invention has a lower stress when being stretched, the mechanical stretching process (i.e., the "zero strain" stretch laminate formation process) is not required for forming stretchable ear panels of disposable garments.

The fiber oriented nonwoven of the present invention is formed from component fibers which are joined together. The component fibers have a primary fiber direction. The fiber oriented nonwoven of the present invention has a Fiber Orientation Ratio within about ±20 degrees from the primary fiber direction (FOR20) of at least about 65%; more preferably at least about 75%; more preferably still at least about 85%. In a more preferred embodiment, the fiber oriented nonwoven additionally has a Fiber Orientation Ratio within about ±10 degrees from the primary fiber direction (FOR10) of at least about 45%; more preferably at least about 60%; more preferably still at least about 70%.

"FOR#" (e.g., FOR10) indicates the ratio of the number of the component fibers whose directions are within about ±# degrees, (e.g., ±10 degrees) from the primary fiber direction to the total number of component fibers. Herein, "primary fiber direction" refers to an average direction of component fibers in the nonwoven layer. The method for measuring the Fiber Orientation Ratio of nonwoven materials is described in the Test Methods section..

In a preferred embodiment, the fiber oriented nonwoven has a Tensile Strength Ratio (TSR) of at least about 15, more preferably at least about 60. The TSR is defined by the following calculation:

$$TSR = TS1 \, / \, TS2 \tag{1}$$

wherein,

TS1 (gf/inch): a tensile strength (TS) at the breaking point in the primary fiber direction; and
TS2 (gf/inch): a tensile strength (TS) at the breaking point in the perpendicular direction which is perpendicular to the primary fiber direction.

Tensile Strength (ST) is measured as the maximum tensile strength value recorded while the fiber oriented nonwoven is stretched at a rate of about 20 inches/min (about 50 cm/min) to its breaking point. The tensile strength of the fiber oriented nonwoven is measured before the fiber oriented nonwoven is joined to other material(s). A preferred method for measuring the tensile strength of nonwoven materials is described in the Test Methods section.

In a preferred embodiment, the fiber oriented nonwoven has a tensile strength of less than about 200 gf/inch (about 80 gf/cm) at 30% elongation to the direction which is perpendicular to the primary fiber direction; more preferably less than about 100 gf/inch (about 40 gf/cm), more preferably still less than about 50 gf/inch (about 20 gf/cm).

The fiber oriented nonwoven may be manufactured from a wide range of component fibers including, e.g., natural fibers (e.g., wool and cotton fibers), synthetic fibers (e.g., polyolefin, polyester, nylon, and rayon fibers), or a mixture of natural fibers and/or synthetic fibers. For ease of manufacture and cost efficiency, the fiber oriented nonwoven is preferably formed from synthetic continuous fibers. More preferably, such synthetic continuous fibers are formed from a polyolefin (e.g., polyethylene and polypropylene) or a polyester. Preferred polyester material includes a polyethylene terephthalate, a polybutylene terephthalate and a polypropylene terephthalate, or mixtures thereof. In a preferred embodiment, the fiber oriented nonwoven additionally includes component fibers formed from the other materials (i.e., non-polyester materials) such as polyolefin and nylon.

In a preferred embodiment, the individual component fibers are formed from a single type of material which is selected from the above materials (i.e., the individual fiber is not made from polyolefin and nylon). Preferably, the component fibers are formed from a polyester, more preferably a polyethylene terepthalate, or one of its relatives which has an average molecular weight from about 5,000 to about 60,000, preferably from about 10,000 to about 40,000, more preferably from about 14,000 to about 23,000. Alternatively, the component fibers may be formed from a mixture of two (or more) materials which are selected from the above materials.

In one embodiment, the component fiber has a bi-component fiber structure formed from two distinct materials of a polyester and a polyolefin. In an alternative embodiment, the component fiber has a bi-component fiber structure formed from two distinct molecular weight materials of one identical material, for example, a polyester. Preferred bi-component fiber structures may include a side-by-side bi-component fiber structure and a sheath-core bi-component fiber structure known in the art. In one embodiment, the component fiber has a bi-component fiber structure having a core of polyester and a sheath of polyolefin.

In a preferred embodiment, the fiber oriented nonwoven has a basis weight of less than about 60 $g/m^2$, and comprises fibers having a fiber diameter of less than about 50 $\mu$m. More preferably, for products such as disposable garment and the like, the fiber oriented nonwoven has a basis weight of from about 3 $g/m^2$ to about 50 $g/m^2$, more preferably from about 10 $g/m^2$ to about 25 $g/m^2$, and a fiber diameter of from about 1 $\mu$m to about 30 $\mu$m, more preferably from about 3 $\mu$m to about 20 $\mu$m.

The component fibers may be joined together by adhesives, thermal bonding, water-jetting, needling/felting, or other methods known in the art to form nonwoven fabrics. In a preferred embodiment, the fiber oriented nonwoven is formed from a nonwoven manufacturing process handling continuous component fibers or filaments known in the art. Preferred manufacturing process are described in, for example, EP 0843036A1 (Kurihara et al.) published on May 20, 1998; U.S. Patent No. 5,312,500, entitled "Non-Woven Fabric and Method and Apparatus for Making The Same", issued to Kurihara et al. on May 17, 1994; Japanese Laid-Open Patent Publication (Kokai) No. H2-269859 published on November 5, 1990; and Japanese Patent Publication (Kokoku) No. S60-25541 published on June 19, 1985.

Preferred nonwoven fabrics which are suitably applicable to the fiber oriented nonwoven are available from Nippon Petrochemicals Co., Ltd., Tokyo, Japan, under Code Nos. MBE8202-3-2; MBE8202-3-1; MBE7711-2; MBE6515-10; and MBE7922-1 which have the following properties.

Table

| Code No. | Basis Weight ($g/m^2$) | FOR10 (%) | FOR20 (%) | TSR at Break Point | TS at 30% elongation |
|---|---|---|---|---|---|
| MBE8202-3-2 | 15 | 77 | 93 | 106 | 10 |
| MBE8202-3-1 | 20 | 82 | 94 | 68 | 20 |
| MBE7711-2 | 21 | 69 | 92 | 76 | 133 |
| MBE6515-10 | 8 | 60 | 85 | 104 | 21 |
| MBE7922-1 | 29 | 58 | 80 | 33 | 157 |

In a preferred embodiment, the disposable garment of the present invention is a disposable pull-on diaper. In the following, such a disposable pull-on diaper will be described in detail by referring to drawings.

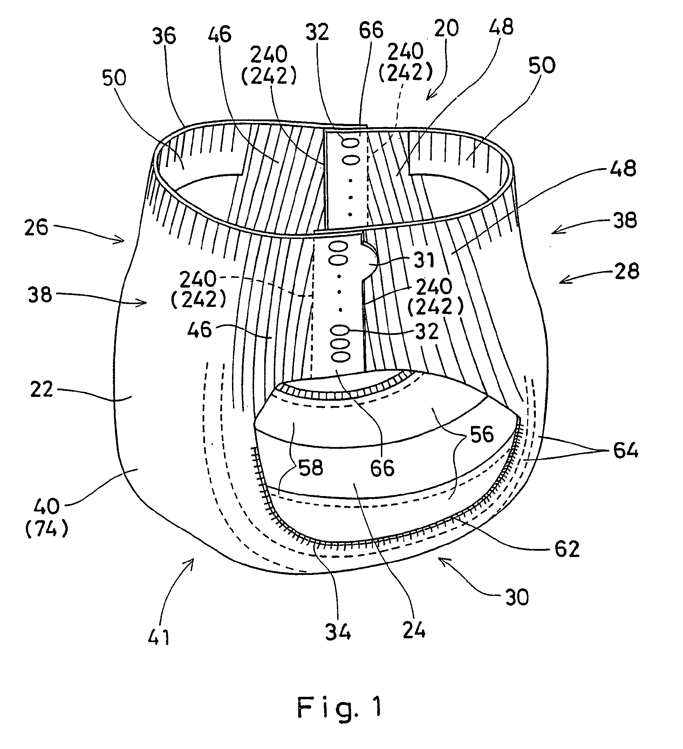

Fig. 1 shows one preferred embodiment of a disposable pull-on garment of the present invention (e.g., a unitary disposable pull-on diaper). Referring to Fig. 1, the disposable pull-on diaper 20 has a front region 26; a back region 28 and a crotch region 30 between the front region 26 and the back region 28. A chassis 41 is provided in the front, back and crotch regions 26, 28 and 30. The chassis 41 includes a topsheet 24 which is preferably liquid pervious, a backsheet 22 which is preferably liquid impervious associated with the topsheet 24, and an absorbent core 25 (not shown in Fig.

1) disposed between the topsheet 24 and the backsheet 22.

The disposable pull-on diaper 20 further includes a pair of front ear panels 46 each extending laterally outward from the corresponding sides of the chassis 41 in the front region 26, and a pair of stretchable back ear panels 48 each extending laterally outward from the corresponding sides of the chassis 41 in the back region 28. Each of the ear panels 46 and 48 has an outermost edge 240 which forms an outermost edge line 242. The pull-on diaper 20 further includes seams 32 each joining the front and back ear panels 46 and 48 along the corresponding edge lines 242 to form the two leg openings 34 and the waist opening 36.

In preferred embodiments, the pull-on diaper 20 includes a chassis layer 40 which generally determines the overall shape of the pull-on diaper 20. In the embodiment shown in Fig. 1, the chassis layer 40 is an outer cover nonwoven layer 74 which covers all of the garment-facing surface of the pull-on diaper 20 to provide the feel and appearance of a cloth garment. The continuous sheet (i.e., the outer cover nonwoven layer 74) defines the front region 26, the back region 28 and the crotch region 30 between the front region 26 and the back region 28. Each of the ear panels 46 and 48 includes a portion of the chassis layer 40. Preferred pull-on diapers which includes a continuous sheet structure are disclosed in U.S. Patent No. 5,569,234 issued to Buell et al. on October 29, 1996.

In a preferred embodiment, at least one of, more preferably both of, the pairs of the ear panels 46 and 48 are elastically stretchable in at least the lateral direction. Herein, "stretchable" refers to materials that are capable of stretching in at least one direction to a certain degree without undue rupture. Herein, "elasticity" and "elastically stretchable" refer to stretchable materials that have the ability to return to approximately their original dimensions after the force that stretched the material is removed. Herein, any material or element described as "stretchable" may also be elastically stretchable unless otherwise provided. The stretchable ear panels 46 and 48 provide a more comfortable and contouring fit by initially conformably fitting the pull-on diaper to the wearer and sustaining this fit throughout the time of wear well past when the pull-on diaper has been loaded with exudates since the ear panels 46 and/or 48 allow the sides of the pull-on diaper to expand and contract.

The ear panels 46 and 48 may be formed by unitary elements of the pull-on diaper 20 (i.e., they are not separately manipulative elements secured to the pull-on diaper 20, but rather are formed from and are extensions of one or more of the various layers of the pull-on diaper). In a preferred embodiment, the ear panels 46 and 48 include at least one unitary element or a continuous sheet (e.g. the chassis layer 40) that forms a part of the chassis 41 and continuously extends into the ear panels 46 and 48.

In a preferred embodiment, the pull-on diaper 20 further includes seam panels 66 each extending laterally outward from each of the ear panels 46 and 48; and tear open tabs 31 each extending laterally outward from the seam panel 66. In a preferred embodiment, each of the seam panels 66 is an extension of the corresponding ear panels 46 and 48, or at least one of the component elements used therein (e.g., the chassis layer 40), or any other combination of the elements. More preferably, each of the tear open tabs 31 is also an extension of the corresponding seam panel 66 or at least one of its component elements used therein (e.g., the chassis layer 40), or any other combination of its elements.

In a preferred embodiment, the corresponding edge portions of the ear panels 46 and 48 are joined through the seam panels 66 in an overlapping manner to make an overlapped seam structure as shown in Fig. 1. Alternatively, the front and back ear panels 46 and 48 can be seamed in a butt seam manner (not shown in Figs.). The bonding of the seams 32 can be performed by any suitable means known in the art appropriate for the specific materials employed in the seam panels 66. Thus, sonic sealing, heat sealing, pressure bonding, adhesive or cohesive bonding, sewing, autogeneous bonding, and the like may be appropriate techniques. Preferably, the seam panels 66 are joined by a predetermined pattern of heat/pressure or ultrasonic welds which withstands the forces and stresses generated on the garment 20 during wear.

A continuous belt 38 is formed by the ear panels 46 and 48, and a part of the chassis 41 about the waist opening 36 as shown in Fig. 1. Preferably, elasticized waist bands 50 are provided in both the front region 26 and the back region 28. The continuous belt 38 acts to dynamically create fitment forces in the pull-on diaper 20 when positioned on the wearer, to maintain the pull-on diaper 20 on the wearer even when loaded with body exudates thus keeping the absorbent core 25 (not shown in Fig. 1) in close proximity to the wearer, and to distribute the forces dynamically generated during wear about the waist thereby providing supplemental support for the absorbent core 25 without binding or bunching the absorbent core 25.

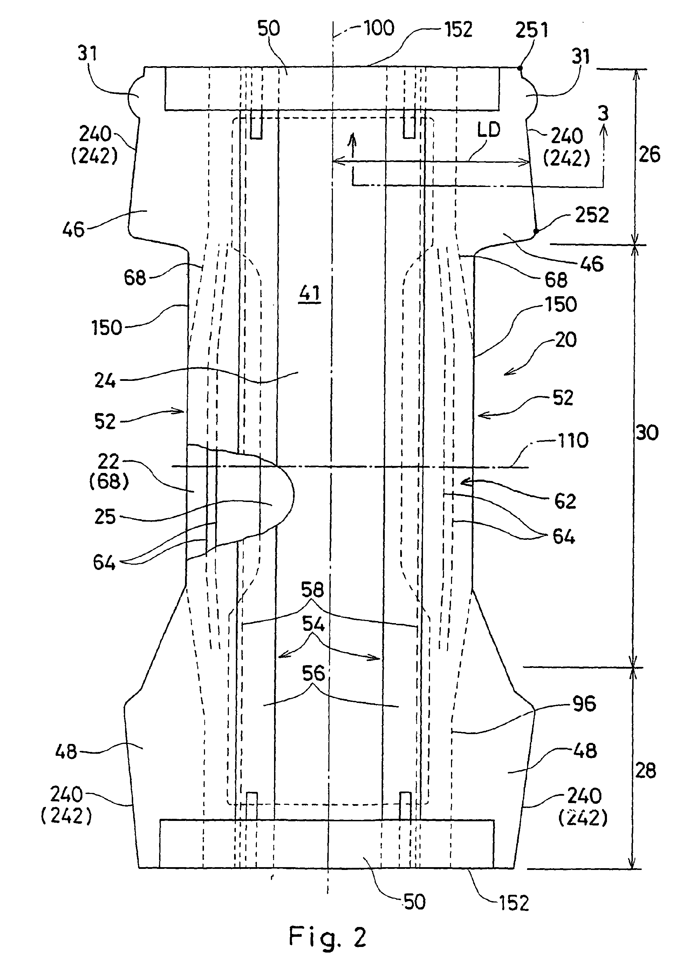

Fig. 2 is a partially cut-away plan view of the pull-on diaper 20 of Fig. 1 in its uncontracted state (except in the ear panels 46 and 48 which are left in their relaxed condition) with the topsheet 24 facing the viewer, prior to the ear panels 46 and 48 being joined together by the seams 32 (shown in Fig. 1). The pull-on diaper 20 has the front region 26, the back region 28 opposed to the front region 26, the crotch region 30 positioned between the front region 26 and the back region 28, and a periphery which is defined by the outer perimeter or edges of the pull-on diaper 20 in which the side edges are designated 150 and 240, and the end edges or waist edges are designated 152. The topsheet 24 has the body-facing surface of the pull-on diaper 20 which is positioned adjacent to the wearer's body during use. The backsheet 22 has the garment-facing surface of the pull-on diaper 20 which is positioned away from the wearer's body.

The pull-on diaper 20 includes the chassis 41 including the liquid pervious topsheet 24, the liquid impervious backsheet 22 associated with the topsheet 24, and the absorbent core 25 positioned between the topsheet 24 and the backsheet 22. The garment 20 further includes the front and back ear panels 46 and 48 extending laterally outward from the chassis 41, the elasticized leg cuffs 52, and the elasticized waistbands 50. The topsheet 24 and the backsheet 22 have length and width dimensions generally larger than those of the absorbent core 25. The topsheet 24 and the backsheet 22 extend beyond the edges of the absorbent core 25 to thereby form the side edges 150 and the waist edges 152 of the garment 20. Preferably, the liquid impervious backsheet 22 is a liquid impervious plastic film 68.

The pull-on diaper 20 also has two centerlines, a longitudinal centerline 100 and a transverse centerline 110. Herein, "longitudinal" refers to a line, axis, or direction in the plane of the pull-on diaper 20 that is generally aligned with (e.g. approximately parallel with) a vertical plane which bisects a standing wearer into left and right halves when the pull-on diaper 20 is worn. Herein, "transverse" and "lateral" are interchangeable and refer to a line, axis or direction which lies within the plane of the pull-on diaper that is generally perpendicular to the longitudinal direction (which divides the wearer into front and back body halves). The pull-on diaper 20 and component materials thereof also have a body-facing surface which faces the skin of wearer in use and a garment-facing surface which is the opposite surface to the body-facing surface.

While the topsheet 24, the backsheet 22, and the absorbent core 25 may be assembled in a variety of well known configurations, exemplary chassis configurations are described generally in U.S. Patent 3,860,003 entitled "Contractible Side Portions for Disposable Diaper" which issued to Kenneth B. Buell on January 14, 1975; and U.S. Patent 5.151.092 entitled "Absorbent Article With Dynamic Elastic Waist Feature Having A Predisposed Resilient Flexural Hinge" which issued to Kenneth B. Buell et al., on September 29, 1992.

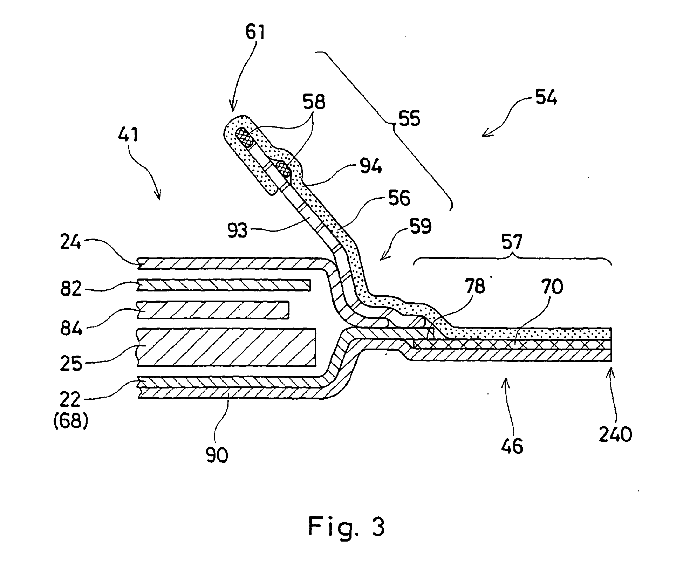

Fig. 3 is a cross-sectional view of a preferred embodiment taken along the section line 3-3 of Fig. 2. The pull-on diaper 20 includes the chassis 41 including the liquid pervious topsheet 24, the liquid impervious backsheet 22 associated with the topsheet 24, and the absorbent core 25 positioned between the topsheet 24 and the backsheet 22. The pull-on diaper 20 further includes the front ear panel 46 extending laterally outward from the chassis 41, and an inner barrier cuff 54. The front ear panel 46 includes an elastic member 70 which provides an elasticity in the ear panel 46. The elastic member 70 includes a plane elastomeric material. Herein, "plane elastomeric material" refers to elastomeric materials which continuously extend in two dimensional directions. Preferably, the elastic member 70 is an elastic laminate which includes such a plane elastomeric material. Preferred plane elastomeric materials include a scrim, a perforated (or apertures formed) film, an elastomeric woven or nonwoven, an elastomeric foam and the like. In a preferred embodiment, each of the front ear panels 46 is formed by a lamination of the elastic laminate 70 and at least a part of a nonwoven layer 56 or the outer cover nonwoven layer 74.

Although Fig. 3 depicts only the structure of one of the front ear panel 46 and a part of the chassis 41, preferably similar structures are also provided in the other front ear panel 46 and the back ear panels 48.

The absorbent core 25 can be any absorbent member which is generally compressible, conformable, non-irritating to the wearer's skin, and capable of absorbing and retaining liquids such as urine and other certain body exudates. The absorbent core 25 may be manufactured in a wide variety of sizes and shapes (e.g., rectangular, hourglass, "T"-shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 25 may vary (e.g., the absorbent core 25 may have varying caliper zones, a hydrophilic gradient, a superabsorbent gradient, or lower average density and lower average basis weight acquisition zones; or may include one or more layers or structures). Further, the size and absorbent capacity of the absorbent core 25 may also be varied to accommodate wearers ranging from infants through adults. However, the total absorbent capacity of the absorbent core 25 should be compatible with the design loading and the intended use of the garment 20.

A preferred embodiment of the garment 20 has an asymmetric, modified hourglass-shaped absorbent core 25 having ears in the front and back waist regions 26 and 28. Other exemplary absorbent structures for use as the absorbent core 25 that have achieved wide acceptance and commercial success are described in U.S. Patent No. 4,610,678 entitled "High-Density Absorbent Structures" issued to Weisman et al. on September 9, 1986; U.S. Patent No. 4,673,402 entitled "Absorbent Articles With Dual-Layered Cores" issued to Weisman et al. on June 16, 1987; U.S. Patent No. 4,888,231 entitled "Absorbent Core Having A Dusting Layer" issued to Angstadt on December 19, 1989; and U.S. Patent No. 4,834,735, entitled "High Density Absorbent Members Having Lower Density and Lower Basis Weight Acquisition Zones", issued to Alemany et al. on May 30, 1989.

The chassis 41 may further include an acquisition/distribution core 84 of chemically stiffened fibers positioned over the absorbent core 25, thereby forming a dual core system as shown in Fig. 3. In a preferred embodiment, the fibers are hydrophilic chemically stiffened cellulosic fibers. Herein, "chemically stiffened fibers" means any fibers which have been stiffened by chemical means to increase stiffness of the fibers under both dry and aqueous conditions. Such means include the addition of chemical stiffening agents which, for example, coat and/or impregnate the fibers. Such means also include the stiffening of the fibers by altering the chemical structure of the fibers themselves, e.g., by cross-linking polymer chains.

The fibers utilized in the acquisition/distribution core 84 can also be stiffened by means of chemical reaction. For example, crosslinking agents can be applied to the fibers which, subsequent to application, are caused to chemically form intrafiber crosslink bonds. These crosslink bonds can increase stiffness of the fibers. Whereas the utilization of intrafiber crosslink bonds to chemically stiffen the fibers is preferred, it is not meant to exclude other types of reactions for chemical stiffening of the fibers.

In the more preferred stiffened fibers, chemical processing includes intrafiber crosslinking with crosslinking agents while such fibers are in a relatively dehydrated, defibrated (i.e. individualized), twisted, curled condition. Suitable chemical stiffening agents include monomeric crosslinking agents including, but not limited to, $C_2$-$C_8$ dialdehydes and $C_2$-$C_8$ monoaldehydes having an acid functionality can be employed to form the cosslinking solution. These compounds are capable of reacting with at least two hydroxyl groups in a single cellulose chain or on proximately located cellulose chains in a single fiber. Such crosslinking agents contemplated for use in preparing the stiffened cellulose fibers include, but are not limited to, glutaraldehyde, glyoxal, formaldehyde, and glyoxylic acid. Other suitable stiffening agents are polycarboxylates, such as citric acid. The polycarboxylic stiffening agents and a process for making stiffened fibers from them are described in U.S. Patent No. 5,190,563, entitled "Process for Preparing Individualized, Polycarboxylic Acid crosslinked Fibers" issued to Herron, on March 2, 1993. The effect of crosslinking under these conditions is to form fibers which are stiffened and which tend to retain their twisted, curled configuration during use in the absorbent articles herein. Such fibers, and processes for making them are cited in the above incorporated patents.

Preferred dual core systems are disclosed in U.S. Patent No. 5,234,423, entitled "Absorbent Article With Elastic Waist Feature and Enhanced Absorbency" issued to Alemany et al., on August 10, 1993; and in U.S. Patent No. 5,147,345, entitled "High Efficiency Absorbent Articles For Incontinence Management" issued to Young, LaVon and Taylor on September 15, 1992. In a preferred embodiment, the acquisition/distribution core 84 includes chemically treated stiffened cellulosic fiber material, available from Weyerhaeuser Co. (U.S.A.) under the trade designation of "CMC". Preferably, the acquisition/distribution core 84 has a basis weight of from about 40 g/m$^2$ to about 400 g/m$^2$, more preferably from about 75 g/m$^2$ to about 300 g/m$^2$.

More preferably, the chassis 22 further includes an acquisition/distribution layer 82 between the topsheet 24 and the acquisition/distribution core 84 as shown in Fig. 3. The acquisition/distribution layer 82 is provided to help reduce the tendency for surface wetness of the topsheet 24. The acquisition/distribution layer 82 preferably includes carded, resin bonded hiloft nonwoven materials such as, for example, available as Code No. FT-6860 from Polymer Group, Inc., North America (Landisiville, New Jersey, U.S.A.), which is made of polyethylene telephthalate fibers of 6 dtex, and has a basis weight of about 43 g/m$^2$. A preferable example for the acquisition/distribution layer 82 and the acquisition/distribution core 84 is disclosed in EP 0797968A1 (Kurt et al.) published on October 1, 1997.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 25 (i.e., to prevent rewet). In a preferred embodiment, the topsheet 24 is formed by the nonwoven layer of the present invention. If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 25. The topsheet 24 can be rendered hydrophilic by treating it with a hydrophobic finishing oil or a surfactant. Suitable methods for the treatment for the topsheet 24 include spraying the topsheet 24 material with the surfactant and immersing the material into the surfactant. A more detailed discussion of such a treatment and hydrophilicity is contained in U.S. Patent No. 4,988,344 entitled "Absorbent Articles with Multiple Layer Absorbent Layers" issued to Reising, et al. on January 29, 1991 and U.S. Patent No. 4,988,345 entitled "Absorbent Articles with Rapid Acquiring Absorbent Cores" issued to Reising on January 29, 1991. Alternatively, the topsheet 24 may be a carded nonwoven material which is formed by hydrophobic component fibers.

In a preferred embodiment, the topsheet 24 is a nonwoven web that can provide reduced tendency for surface wetness; and consequently facilitate maintaining urine absorbed by the core 25 away from the user's skin, after wetting. One of the preferred topsheet materials is a thermobonded carded web which is available as Code No. P-8 from Fiberweb North America, Inc. (Simpsonville, South Carolina, U.S.A.). Another preferred topsheet material is available as Code No. S-2355 from Havix Co., Japan. This material is a bi-layer composite material, and made of two kinds of synthetic surfactant treated bi-component fibers by using carding and air-through technologies. Yet another preferred topsheet material is a thermobonded carded web which is available as Code No. Profleece Style 040018007 from Amoco Fabrics, Inc. (Gronau, Germany).

Another preferred topsheet 24 includes an apertured formed film. Apertured formed films are preferred for the topsheet 24 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3,929,135, entitled "Absorptive Structures Having Tapered Capillaries", issued to Thompson on December 30, 1975; U.S. Patent No. 4,324,246 entitled "Disposable Absorbent Article Having A Stain Resistant Topsheet", issued to Mullane, et al. on April 13, 1982; U.S. Patent No. 4,342,314 entitled "Resilient Plastic Web Exhibiting Fiber-Like Properties", issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", issued to Ahr et al. on July 31, 1984; and U.S. 5,006,394 "Multilayer Polymeric Film" issued to Baird on April 9, 1991.

In a preferred embodiment, the backsheet 22 is the liquid impervious film 68 as shown in, for example, Fig. 3. Preferably, the liquid impervious film 68 longitudinally extends in the front, back and crotch regions 26, 28 and 30. More preferably, the liquid impervious film 68 does not laterally extend into the at least one of the ear panels 46 or 48. The liquid impervious film 68 has a body-facing surface and an garment-facing surface opposing the body-facing surface. The liquid impervious film 68 is impervious to liquids (e.g., urine) and is preferably manufactured from a thin plastic film. Preferably, the liquid impervious film 68 has a water resistance of at least about 200 mmH$_2$O, more preferably at least about 400 mmH$_2$O. In a preferred embodiment, the liquid impervious film 68 has a water resistance of about 600 mmH$_2$O. However, more preferably the plastic film permits vapors to escape from the garment 20. In a preferred embodiment, a microporous polyethylene film is used for the liquid impervious film 68. A suitable microporous polyethylene film is manufactured by Mitsui Chemicals, Inc., Nagoya, Japan and marketed in the trade as PG-P. In a preferred embodiment, a disposable tape (not shown in Figs.) is additionally joined to the outer surface of the backsheet 22 to provide a convenient disposal after soiling. A preferred disposal tape (or device) for pull-on garments is disclosed in International Publication No. WO 94/09736 (Rollag et al.) published on May 11, 1994.

A suitable material for the liquid impervious film 68 is a thermoplastic film having a basis weight of from about 10 g/m$^2$ to about 45 g/m$^2$. Preferably, the thermoplastic film has a basis weight of from about 20 g/m$^2$ to about 40 g/m$^2$. In a preferred embodiment, the thermoplastic film has a basis weight of about 35 g/m$^2$.

The outer cover nonwoven layer 74 (i.e., the chassis layer 40) is joined with the garment-facing surface of the liquid impervious film 68 to form a composite laminate. Preferably, the outer cover nonwoven layer 74 is formed by the fiber oriented nonwoven layer of the present invention. The outer cover nonwoven layer 74 preferably covers all of the garment-facing surface of the pull-on diaper 20 to provide the feel and appearance of a cloth garment. The outer cover nonwoven layer 74 may be joined to the liquid impervious film 68 by any suitable attachment means known in the art. For example, the outer cover nonwoven layer 74 may be secured to the liquid impervious film 68 by a uniform continuous layer of adhesive, a patterned layer of adhesive, or an array of separate lines, spirals, or spots of adhesive. Suitable adhesives include a hotmelt adhesive obtainable from Nitta Findley Co., Ltd., Osaka, Japan as H-2128, and a hotmelt adhesive obtainable from H.B. Fuller Japan Co., Ltd., Osaka, Japan as JM-6064.

In a preferred embodiment, the outer cover nonwoven layer 74 is formed by a nonwoven material which is available from Nippon Petrochemicals Co., Ltd., Tokyo, Japan, under Code Nos. MBE8202-3-2; MBE8202-3-1; MBE7711-2; MBE6S1S-10; and MBE7922-1.

The backsheet 22 is positioned adjacent the garment-facing surface of the absorbent core 25 and is preferably joined thereto by any suitable attachment means known in the art. Specifically, the body-facing surface of the liquid impervious film 68 may be secured to the absorbent core 25 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota, U.S.A., and marketed as HL-13S8J. An example of a suitable attachment means including an open pattern network of filaments of adhesive is disclosed in U.S. Patent No. 4,573,986 entitled "Disposable Waste-Containment Garment", which issued to Minetola et al. on March 4, 1986. Another suitable attachment means including several lines of adhesive filaments swirled into a spiral pattern is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may include heat bonds, pressure bonds, ultrasonic bonds, dynamic mechanical bonds, or any other suitable attachment means or combinations of these attachment means as are known in the art.

In an alternative embodiment, the absorbent core 25 is not joined to the backsheet 22, and/or the topsheet 24 in order to provide greater stretchability in the front region 26 and the back region 28.

The pull-on diaper 20 further includes elasticized leg cuffs 52 for providing improved containment of liquids and other body exudates. The elasticized leg cuffs 52 may include several different embodiments for reducing the leakage of body exudates in the leg regions. (The leg cuffs can be and are sometimes also referred to as leg bands, side flaps, barrier cuffs, elastic cuffs or gasketing cuffs.) U.S. Patent 3,860,003 entitled "Contractable Side Portions for Disposable Diaper" issued to Buell on January 14, 1975, describes a disposable diaper which provides a contractible leg opening having a side flap and one or more elastic members to provide an elasticized leg cuff. U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987; and U.S. Patent 4,795,454 entitled "Absorbent Article Having Leakage-Resistant Dual Cuffs" issued to Dragoo on January 3, 1989, describe disposable diapers having dual cuffs including a gasketing cuff and a barrier cuff. In a preferred embodiment, such barrier cuffs or "stand-up" elasticized flaps includes the fiber oriented nonwoven layer of the present invention.

While each elasticized leg cuff 52 may be configured so as to be similar to any of the leg bands, side flaps, barrier cuffs, or elastic cuffs described above, it is preferred that the elasticized leg cuff 52 includes an elastic gasketing cuff 62 with one or more elastic strands 64 as shown in Fig. 2, an inner barrier cuff 54 which includes a nonwoven layer 56, and spacing elastic members 58 which are described in the above-referenced U.S. Patent Nos. 4,695,278 and 4,795,454.

Referring again to Fig. 3, the inner barrier cuff 54 includes a channel portion 55 having a proximal edge 59 joined to the topsheet 24, and a distal edge 61 which is away from the body-facing surface of the topsheet 24. The inner barrier cuff 54 further includes a flap portion 57 which extends laterally outward from the proximal edge 59 into at least a part of the ear panel 46. In the embodiment shown in Fig. 3, the flap portion 57 extends to the outermost edge 240 of the ear panel 46.

The distal edge 61 of the inner barrier cuff 54 is that part of the inner barrier cuff 54 which is spaced away from the body-facing surface of the topsheet 24 when the pull-on diaper 20 is being worn. The proximal edge 59 is that part of the inner barrier cuff 54 which is joined to the topsheet 24. The proximal edge 59 is preferably located laterally inboard of the outermost edge 240 of the pull-on diaper 20.

In a preferred embodiment, the barrier cuff 54 is formed by two nonwoven layers 93 and 94 as shown in Fig. 3. One of such two nonwoven layers is the fiber oriented nonwoven layer 94 of the present invention, and the other works as a water resistant layer 93 for increasing the water resistant value of the barrier cuff 54. The fiber oriented nonwoven layer 94 is folded back at the distal edge 61 onto the water resistant layer 93 to enclose the spacing elastic members 58 as shown in Fig. 3.

Any materials which can increase the water resistant value of the barrier cuff 54 can be used for the water resistant layer 93. Preferred materials for the water resistant layer 93 includes nonwoven materials and plastic film materials. Preferably, the water resistant layer 93 has a water resistance of at least about 50 mmH$_2$O, more preferably at least about 70 mmH$_2$O. In a preferred embodiment, the water resistant layer 93 has a water resistance of about 100 mmH$_2$O.

The water resistant layer 93 is joined or attached to the fiber oriented nonwoven layer 94 to form a composite laminate in the barrier cuff 54. Any attachment means known in the art for attaching one material to another can be used. Preferred attachment means includes adhesive bonds, heat bonds, pressure bonds, ultrasonic bonds, dynamic mechanical bonds, or combinations of these means. In a preferred embodiment, a pressure bond is used for attaching the water resistant layer 93 to the fiber oriented nonwoven layer 94.

In a preferred embodiment, the nonwoven layer 93 (i.e., the water resistant layer) is formed by a meltblown nonwoven material of synthetic fibers. A preferred meltblown nonwoven material is made of fine fibers of a polypropylene (PP). Preferably, the meltblown nonwoven layer 93 has a water resistance value of at least about 200 mmH$_2$O, more preferably, at least about 300 mmH$_2$O.

In a more preferred embodiment, the meltblown nonwoven layer 93 has an embossment pattern. As used herein, the term "embossment" is used herein to describe a material which has at least a portion where the material was pressed and heated before it is used in final products. As used herein, the term "pattern" is used herein to describe the embossed portion(s) of the material has a predetermined arrangement. In preferred embodiments, the predetermined arrangement comprises a plurality of discrete embossed portions. The embossment pattern can be any pattern known in the art such as spots, lattice, diagonal, interrupted-line and the like. Preferably, the embossment pattern is formed by passing the material between a pair of heated bonding rolls (i.e., embossment rolls) which have a corresponding pattern to a desired embossment pattern. The material is compressed and heated by the bonding rolls in accordance with the pattern on the rolls to create the embossment pattern in which the particular filaments and/or fibers of the material are bonded. The embossment pattern can increase the strength of the material against the friction which is applied by the skin of wearer when the article is used.

The embossment ratio of the material is defined by the ratio of the total area of embossing portion to the total area of non-embossing portion of the heated bonding rolls. Preferably, the embossment ratio for the meltblown nonwoven layer 93 is at least 0.5 %, more preferably from about 1% to about 35 %.

Preferred materials for the fiber oriented nonwoven layer 94 are available from Nippon Petrochemicals Co., Ltd., Tokyo, Japan, under Code Nos. MBE8202-3-2; MBE8202-3-1; MBE7711-2; MBE6515-10; and MBE7922-1.

In an alternative embodiment (not shown in Figs.), the barrier cuff 54 can be formed only by a single layer 94 of the fiber oriented nonwoven material of the present invention (i.e., without the water resistant layer 93). In this embodiment, the fiber oriented nonwoven layer 94 is folded back upon itself at the distal edge 61 to enclose the spacing elastic members 58. The fiber oriented nonwoven layer 94 preferably has a water resistance value of at least about 50 mmH$_2$O, and an average bending force value of less than about 20 mg cm$^2$/cm. More preferably, the fiber oriented nonwoven layer 94 has a water resistance value of at least about 70 mmH$_2$O.

In a further alternative embodiment (not shown in Figs.), the barrier cuff 54 can be formed by a two layered structure formed by the fiber oriented nonwoven material of the present invention (i.e., without the water resistant layer 93). In this embodiment, the fiber oriented nonwoven layer 94 is folded back upon itself at the distal edge 61 to enclose the spacing elastic members 58, and are attached together by itself by an attachment means to form the two layered structure. Any attachment means known in the art can be used. Preferred attachment means includes adhesive bonds, heat bonds, pressure bonds, ultrasonic bonds, dynamic mechanical bonds, or combinations of these means. In a preferred embodiment, a pressure bond is used for forming the two layered structure. Preferably, the two layered structure formed by the fiber oriented nonwoven layer 94 has a water resistance value of at least about 200 mmH$_2$O.

The expected water resistance value of the fiber oriented nonwoven layer 94 can be achieved by using a polyolefin material for the component fibers of the fiber oriented nonwoven layer 94. A preferred polyolefin material for the component fibers is a polypropylene material.

Referring again to Fig. 2, the pull-on diaper 20 preferably further includes an elasticized waistband 50 that provides improved fit and containment. The elasticized waistband 50 is that portion or zone of the pull-on diaper 20 which is intended to elastically expand and contract to dynamically fit the wearer's waist. The elasticized waistband 50 preferably extends longitudinally outwardly from the waist edge of the pull-on diaper 20 toward the waist edge of the absorbent core 25. Preferably, the pull-on diaper 20 has two elasticized waistbands 50, one positioned in the back region 28 and one positioned in the front region 26, although other pull-on garment embodiments can be constructed with a single elasticized waistband. The elasticized waistband 50 may be constructed in a number of different configurations including those described in U.S. Patent 4,515,595 entitled "Disposable Diapers with Elastically Contractible Waistbands" issued to Kievit et al. on May 7, 1985 and the above referenced U.S. Patent 5,151,092 issued to Buell. The waistbands 50 may include the fiber oriented nonwoven of the present invention.

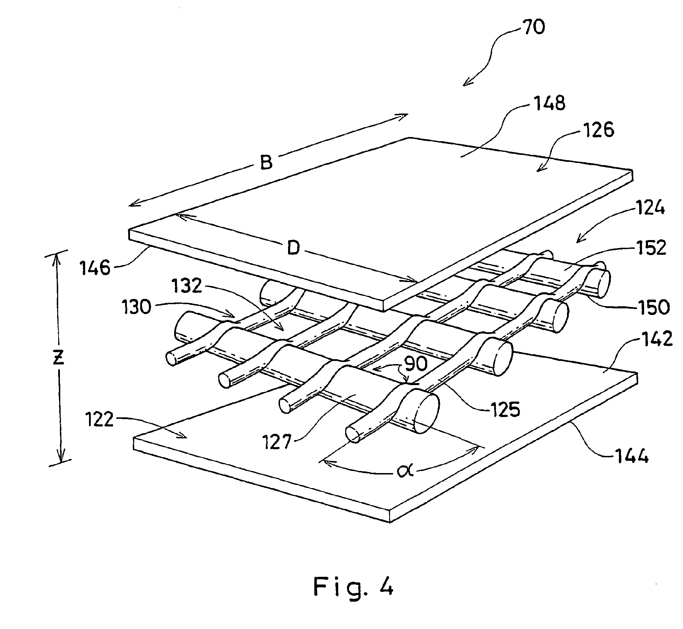

The ear panel 46 includes the elastic laminate 70 as shown in Fig. 3. The elastic laminate 70 includes the elastomeric material layer 124 (not shown in Fig. 3 but Fig. 4) which preferably extends laterally outward from the chassis 41 to provide good fitness by generating the optimal retention (or sustained) force at the waist and side areas of the wearer. Preferably, the elastomeric material layer 124 is stretchable in at least the lateral direction to generate a retention (or sustained) force that is optimal to prevent the pull-on diaper 20 from drooping, sagging, or sliding down from its position on the torso without causing the red marking on the skin of the wearer. In a preferred embodiment, each of the ear panels 46 and 48 includes the elastomeric material layer 124.

The elastic laminate 70 is operatively joined to the nonwoven layer 56 and/or the outer cover nonwoven layer 74 in the ear panel 46 to form a laminate. In a preferred embodiment, the elastic laminate 70 is operatively joined to both of the nonwoven layer 56 and the outer cover nonwoven layer 74 in a substantially untensioned (zero strain) condition.

Preferably, the elastic laminate 70 can be operatively joined to the nonwoven layer 56 and/or the outer cover nonwoven layer 74, by using either an intermittent bonding configuration or a substantially continuous bonding configuration. Herein, "intermittently" bonded laminate means a laminate wherein the plies are initially bonded to one another at discrete spaced apart points or a laminate web wherein the plies are substantially unbonded to one another at discrete spaced apart areas. Conversely, a "substantially continuously" bonded laminate web means a laminate web wherein the plies are initially bonded substantially continuously to one another throughout the areas of interface. It is preferred that the stretch laminate be bonded over all or a significant portion of the stretch laminate so that the nonwoven layers 56 and 74 elongate or draw without causing rupture, and the layers of the era panel 46 are preferably bonded in a configuration that maintains all of the layers of the laminate in relatively close adherence to one another after the incremental mechanical stretching operation. Consequently, the elastic panel members and the other plies of the stretch laminate are preferably substantially continuously bonded together using an adhesive. In a particularly preferred embodiment, the adhesive selected is applied with a control coat spray pattern at a basis weight of about 7.0 grams/m$^2$. The adhesive pattern width is about 6.0 cm. The adhesive is preferably an adhesive such as is available from Nitta Findley Co., Ltd., Osaka, Japan, under the designation H2085F. Alternatively, the elastic panel member and any other components of the stretch laminates may be intermittently or continuously bonded to one another using heat bonding, pressure bonding, ultrasonic bonding, dynamic mechanical bonding, or any other method as is known in the art.

By operatively joining the elastic laminate 70 to the nonwoven layer 56 and the outer cover nonwoven layer 74, the elastically stretchable ear panel 46 can be formed without the mechanical stretching process (i.e., the "zero strain" stretch laminate formation process). This configuration allows the ear panel 46 to be elastically stretchable in the lateral direction.

The elastic laminate 70 is preferably joined to, more preferably directly secured to the respective edges 78 of the liquid impervious film 68 through an adhesive. Preferably, while liquid impervious film 68 longitudinally extends in the front, back and crotch regions 26, 28 and 30, it does not laterally extend into at least one of, preferably each of the stretchable ear panels 46 and 48. In a more preferred embodiment, the elastic laminate 70 is joined to the respective edges 78 of the liquid impervious film 68 at the garment-facing surface as shown in Fig. 3. In an alternative embodiment, the elastic laminate 70 may be joined to the respective edges 78 of the liquid impervious film 68 at the body-facing surface (not shown in Fig. 3). The elastic laminate 70 may be joined to the respective edges 78 of the liquid impervious film 68 by any bonding means known in the art which include adhesive bonds, heat bonds, pressure bonds, ultrasonic bonds, dynamic mechanical bonds, or combinations of these attachment means.

Referring to Fig. 4, the elastic laminate 70 includes the elastomeric material layer 124 having a first surface 150 and a second surface 152 opposing the first surface 150, and a first coverstock layer 122 which is joined to the first surface 150 of the elastomeric material layer 124. In a preferred embodiment, the first coverstock layer 122 is joined to the first surface 150 of the elastomeric material layer 124 by an adhesive (not shown in Fig. 4). More preferably, the elastic laminate 70 further includes a second coverstock layer 126 which is joined to the second surface 152 of the elastomeric material layer 124 by an adhesive (not shown in Fig. 4). In a preferred embodiment, at least one, more preferably both of the first and second coverstock layers 122 and 126 is formed by the fiber oriented nonwoven layer of the present invention. Preferred nonwoven materials for the first and second coverstock layers 122 and 126 are available from Nippon Petrochemicals Co., Ltd., Tokyo. Japan, under Code Nos. MBE8202-3-2; MBE8202-3-1; MBE7711-2; MBE6515-10; and MBE7922-1.

The elastomeric material layer 124 may be formed in a wide variety of sizes, forms and shapes. In a preferred embodiment, the elastomeric material layer 124 is in the form of a continuous plane layer. Preferred forms of continuous plane layer include a scrim, a perforated (or apertures formed) film, an elastomeric woven or nonwoven, and the like. The continuous plane layer may take any shape which can be suitably provided in the ear panels. Preferred shapes of continuous plane layer include a quadrilateral including a rectangle and a square, a trapezoid, and the other polygons. In an alternative embodiment, the elastomeric material layer 124 is in the form of discrete strands (or strings) which are not connected each other.

Elastomeric materials which have been found to be especially suitable for the elastomeric material layer 124 are styrenic block copolymer based scrim materials, perforated (or apertured) elastic films, preferably with a thickness of from about 0.05 mm to about 1.0 mm (0.002 inch - 0.039 inch). Other suitable elastomeric materials for the elastomeric material layer 124 include "live" synthetic or natural rubber, other synthetic or natural rubber foams, elastomeric films (including heat shrinkable elastomeric films), elastomeric woven or nonwoven webs, elastomeric composites, or the like.

In the preferred embodiment shown in Fig. 4, the elastomeric material layer 124 is an elastomeric scrim which has a plurality of first strands 125 and a plurality of second strands 127. The plurality of first strands 125 intersect the plurality of second strands 127 at nodes 130 at a predetermined angle $\alpha$, forming a net-like open structure having a plurality of apertures 132. Each aperture 132 is defined by at least two adjacent first strands and at least two adjacent second strands, so that the apertures 132 are substantially rectangular in shape. Other configurations of the apertures 132, such as parallelograms, squares, or circular arc segments, can also be provided. Preferably, the first and second strands 125 and 127 are substantially straight and substantially parallel to one another. Preferably, the first strands 125 intersect the second strands 127 at nodes 130 such that the angle $\alpha$ is about 90 degrees. The first and second strands 125 and 127 are preferably joined or bonded at nodes 90.

A preferred elastomeric scrim 124 is manufactured by the Conwed Plastics Company (Minneapolis, Minn., U.S.A.) under the designation XO2514. This material has about 12 elastic strands per inch in the structural direction B (i.e., the first strands 125) and about 7 elastic strands per inch in the structural direction D (i.e., the second strands 127).

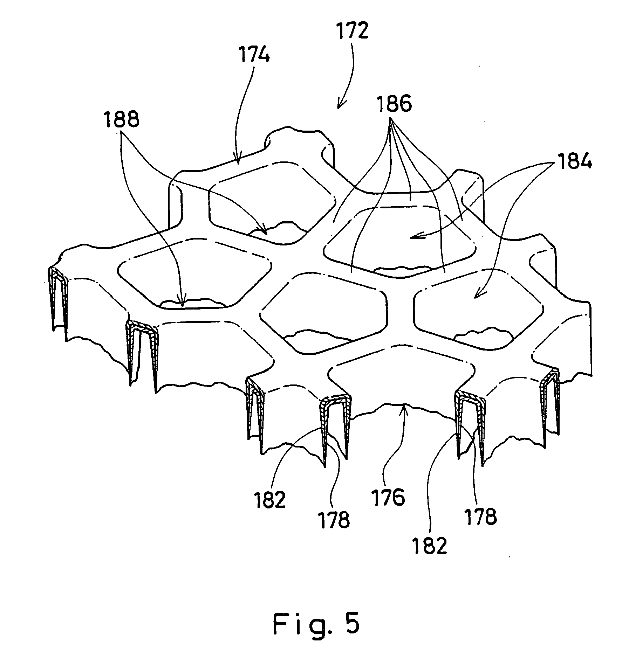

In an alternative preferred embodiment, the elastomeric material layer 124 is a porous, macroscopically-expanded, three-dimensional elastomeric web. Referring to Fig. 5, the porous, macroscopically-expanded, three-dimensional elastomeric web 172 has a continuous first surface 174 and a discontinuous second surface 176 opposing the first surface 174. The elastomeric web 172 preferably comprises a formed film interconnecting member 186 including at least two polymeric layers 178 and 182. The first layer 178 is substantially elastic and the second layer 182 is substantially less elastic than the first layer 178. At least one of the two polymeric layers 178 and 182 is formed from a polystyrene thermoplastic elastomer. The elastomeric web 172 exhibits a multiplicity of primary apertures 184 in the first surface 174 of the web 172. The primary apertures 184 are defined in the plane of the first surface 174 by a continuous network of the interconnecting member 186. The interconnecting member 186 exhibits an upwardly concave-shaped cross-section along its length. The interconnecting member 186 also forms secondary apertures 188 in the plane of the second surface 176 of the web 172. The apertures 184 and 188 may take any shape. A preferred elastomeric web is disclosed in International Publication No. WO 98/3716 (Curro et al.), published on August 27, 1998. A preferred porous elastomeric material for the elastomeric material layer is available from Tredegar Film Products under the designation X-25007.

In the embodiment shown in Fig. 4, the first coverstock layer 122 has an inner surface 142 and an outer surface 144. The inner surface 142 of the first coverstock layer 122 is the surface that is positioned facing the elastomeric material layer 124. The second coverstock layer 126 also has an inner surface 146 and an outer surface 148. The inner surface 146 of the second coverstock layer 126 is the surface that is positioned facing the elastomeric material layer 124. The first and second surfaces 150 and 152 of the elastomeric material layer 124 are substantially parallel with the planes of the first and second coverstock layers 122 and 126. The first surface 150 is that planar surface of the elastomeric material layer 124 that is most closely adjacent with the inner surface 142 of first coverstock layer 122. The second surface 152 is that planar surface of elastomeric material layer 124 that is most closely adjacent to the inner surface 146 of the second coverstock layer 126.

Test Methods

1. Test Method for Fiber Orientation

The following method is used to determine the Fiber Orientation Ratio (FOR) of nonwoven material.

A sample nonwoven fabric (or layer) is placed on a specimen stub. The sample nonwoven fabric is fixed on the specimen stub at a flat condition so that the primary fiber direction (to be defined hereinafter) of the sample nonwoven fabric can be roughly aligned with the longitudinal direction of the photograph to be taken. A scanning electron microscope (SEM) is used to take a photograph at 50X magnification. A preferred SEM is available from Japan Electron Optics Laboratory (JEOL) Ltd., Tokyo, Japan, under Code No. JSM-5310.

The following analysis is conducted on the photograph by using a digitizer. A preferred digitizer is available from Graphtec Co., Ltd.; Tokyo, Japan, under Code No. KD9600. A photograph is placed on the digitizer. A square area (500 $\mu$m x 500 $\mu$m) of the sample nonwoven fabric is chosen at will in the photograph on the digitizer. The both ends of every component fiber which can be seen in the square area are manually identified by an operator and the coordinates thereof are detected and recorded by the digitizer. This work is conducted on three different square areas (each having 500 $\mu$m x 500 $\mu$m) which are chosen at will in the photograph to obtain coordinate data on the all fibers in the three different square areas. The orientation angle of each fiber is calculated based on the coordinate data. The primary fiber direction of the sample nonwoven fabric is determined by the average orientation angle, which is an average value of the all orientation angle data obtained from the three different square areas.

The Fiber Orientation Ratio within about $\pm$10 degrees (FOR 10) is determined by the following calculation:

$$\mathrm{FOR10 = NF10 \ / \ TNF \ x \ 100} \qquad (2)$$

wherein,
NF10: the number of fibers which have orientation angles within about $\pm$10 degrees from the primary fiber direction; and
TNF: the total number of fibers measured within the three different square areas.

Similarly, the Fiber Orientation Ratio within about $\pm$20 degrees (FOR20) is determined by the following calculation:

$$\mathrm{FOR20 = NF20 \ / \ TNF \ x \ 100} \qquad (3)$$

wherein,
NF20: the number of fibers which have orientation angles within about $\pm$20 degrees from the primary fiber direction.

2. Test Method for Tensile Strength

The following method is preferably used to measure the tensile strength of materials.

A tensile tester is prepared. The tensile tester has an upper jaw and a lower jaw which is located below the upper jaw. The upper jaw is movable and is connected to an extension force measuring means. The lower jaw is fixed in the tester. A test specimen (i.e., a nonwoven fabric to be measured) which has about 2.5 cm (about 1 inch) in width and about 10.2 cm (about 4 inches) in length is prepared and clamped with the upper jaw and the lower jaw so that the effective specimen length (L) (i.e., gauge length) is about 5.1 cm (about 2 inch). An extension force is continuously applied to the test specimen through the upper jaw at a cross-head speed of about 50 cm (about 20 inches) per minute, until the test specimen is physically broken. The applied extension force is recorded by a recorder (e.g., a computer system). The tensile strength at the breaking point is determined at the maximum tensile strength value. A tensile tester suitable for use is available from Instron Corporation (100 Royall Street, Canton, MA02021, U.S.A.) as Code No. Instron 5564.

**Claims**

1. A disposable garment (20) having a front region (26), a back region (28) and a crotch region (30) between the front region (26) and the back region (28), comprising:

   a chassis (41) provided in the front, back and crotch regions (26, 28, 30), the chassis (41) comprising a topsheet (24), a backsheet (22) associated with the topsheet (24), and an absorbent core (25) disposed between the topsheet (24) and the backsheet (22), the backsheet (22) having a garment-facing surface; and
   an outer cover (40) disposed on the garment-facing surface of the backsheet **characterized in that** the disposable garment is a pull-on diaper and wherein the outer cover (40) includes a first fiber oriented nonwoven layer having a Fiber Orientation Ratio as defined herein within about $\pm 20$ degrees from a primary fiber direction of at least about 65%.

2. A disposable garment (20) having a front region (26), a back region (28) and a crotch region (30) between the front region (26) and the back region (28), comprising:

   a chassis (41) provided in the front, back and crotch regions (26, 28, 30), the chassis (41) comprising a topsheet (24), a backsheet (22) associated with the topsheet (24), and an absorbent core (25) disposed between the topsheet (24) and the backsheet (22);
   at least one pair of stretchable ear panels (46, 48) extending laterally outward from the chassis (41) in the front or back region (26, 28); and
   a barrier cuff (54) includes (a) a channel portion (55) having a proximal edge (59) joined to the topsheet (24) and a the distal edge (61) which is away from the topsheet (24), and (b) a flap portion (57) which extends laterally outward from the proximal edge (59) into at least a part of the ear panel (46, 48);

   **characterized in that** the disposable garment is a pull-on diaper and wherein at least the flap portion includes a second fiber oriented nonwoven layer having a Fiber Orientation Ratio as defined herein within about $\pm 20$ degrees from a primary fiber direction of at least about 65%.

3. The disposable garment (20) of Claim 1, further comprising at least one pair of stretchable ear panels (46, 48) extending laterally outward from the chassis (41) in the front or back region (26, 28), wherein the first fiber oriented nonwoven layer extends laterally outward from the chassis (41) into the part of the ear panel (46, 48).

4. The disposable garment (20) of Claim 2, wherein the second fiber oriented nonwoven layer extends laterally outward from the chassis (41) into the part of the ear panel (46, 48).

5. The disposable garment (20) of Claim 3 or 4, wherein the ear panel (46, 48) further includes an elastomeric material (124) joined to the first or second fiber oriented nonwoven layer thereby forming an elastically stretchable laminate (70).

6. The disposable garment (20) of Claim 1, wherein the backsheet (22) further includes a first water resistant layer joined to the first fiber oriented nonwoven layer, and wherein the first water resistant layer has a water resistance of at least about 200 $mmH_2O$.

7. The disposable garment (20) of Claim 2, wherein the channel portion (55) includes a second water resistant layer (93) joined to the second fiber oriented nonwoven layer (94), an wherein the second water resistant layer (93) has a water resistance of at least about 50 $mmH_2O$.

8. The disposable garment (20) of Claim 6 or 7, wherein the water resistant layer is joined to the fiber oriented nonwoven layer through a heat/pressure bond or an adhesive.

9. The disposable garment (20) of Claim 1, wherein the first fiber oriented nonwoven layer has a Tensile Strength Ratio of at least about 15.

10. A composite laminate (70), comprising:

a fiber oriented nonwoven layer having a first surface and a second surface opposing the first surface, the fiber oriented nonwoven layer being formed from component fibers having a primary fiber direction; **characterized in that**
a water resistant layer is joined to the first surface of the fiber oriented nonwoven layer, the water resistant layer having a water resistance of at least about 50 mmH$_2$O; and

wherein the fiber oriented nonwoven layer has a Fiber Orientation Ratio as defined herein within about ±20 degrees from a primary fiber direction of at least about 65%.

**Patentansprüche**

1. Einwegwäschestück (20) mit einer vorderen Region (26), einer hinteren Region (28) und einer Schrittregion (30) zwischen der vorderen Region (26) und der hinteren Region (28), welches Folgendes aufweist:

ein Chassis (41), das in den vorderen, hinteren und Schrittregionen (26, 28, 30) bereitgestellt ist, wobei das Chassis (41) eine obere Lage (24), eine untere Lage (22), die mit der oberen Lage (24) verbunden ist, und einen absorbierenden Kern (25), der zwischen der oberen Lage (24) und der unteren Lage (22) angeordnet ist, umfasst,

wobei die hintere Lage (22) eine auf die Wäsche gerichtete Oberfläche aufweist; und
eine äußere Abdeckung (40), die an der auf die Wäsche gerichteten Oberfläche der unteren Lage angeordnet ist, **dadurch gekennzeichnet, dass** das Einwegwäschestück eine Anziehwindel ist und wobei die äußere Abdeckung (40) eine erste Vliesschicht mit orientierter Faser mit einem Faserorientierungsverhältnis wie hierin definiert innerhalb von etwa ±20 Grad von einer primären Faserrichtung von mindestens etwa 65 % einschließt.

2. Einwegwäschestück (20) mit einer vorderen Region (26), einer hinteren Region (28) und einer Schrittregion (30) zwischen der vorderen Region (26) und der hinteren Region (28), das Folgendes umfasst:

ein Chassis (41), das in den vorderen, hinteren und Schrittregionen (26, 28, 30) des Chassis (41) bereitgestellt ist und das eine obere Lage (24), eine untere Lage (22), die mit der oberen Lage (24) verbunden ist, und einen absorbierenden Kern (25), der zwischen der oberen Lage (24) und der unteren Lage (22) angeordnet ist, umfasst; mindestens ein Paar streckbarer Flügelfelder (46, 48), die lateral von dem Chassis (41) nach außen verlaufen, in der vorderen oder hinteren Region (26, 28); und
ein Sperrbündchen (54) schließt (a) einen Kanalabschnitt (55) mit einem proximalen Rand (59); der an die obere Lage (24) gefügt ist, und einem distalen Rand (61), der abseits von der oberen Lage (24) angeordnet ist, und (b) einen Laschenabschnitt (57), der lateral vom proximalen Rand (59) in mindestens einen Teil des Ohrfelds (46, 48) nach außen verläuft;

**dadurch gekennzeichnet, dass** das Einwegwäschestück eine Anziehwindel ist und wobei mindestens der Laschenabschnitt eine Vliesschicht mit einer zweiten Faserorientierung aufweist, die ein Faserorientierungsverhältnis wie hierin definiert innerhalb von etwa ±20 Grad von einer primären Faserrichtung von mindestens etwa 65 % aufweist.

3. Einwegwäschestück (20) nach Anspruch 1, das ferner mindestens ein Paar streckbare Flügelfelder (46, 48), die lateral vom Chassis (41) nach außen verlaufen, in der vorderen oder hinteren Region (26, 28) umfasst, wobei die erste Vliesschicht mit orientierter Faser lateral vom Chassis (41) in den Teil des Flügelfelds (46, 48) verläuft.

4. Einwegwäschestück (20) nach Anspruch 2, wobei die zweite Vliesschicht mit orientierter Faser lateral vom Chassis (41) nach außen in den Teil des Flügelfelds (46, 48) verläuft.

5. Einwegwäschestück (20) nach Anspruch 3 oder 4, wobei das Flügelfeld (46, 48) ferner ein elastomeres Material (124) einschließt, das an die erste oder zweite Vliesschicht mit orientierter Faser angefügt ist, wodurch ein elastisch streckbares Laminat (70) gebildet wird.

6. Einwegwäschestück (20) nach Anspruch 1, wobei die untere Lage (22) ferner eine erste wasserbeständige Schicht einschließt, die an die erste Vliesschicht mit orientierter Faser gefügt ist, und wobei die erste wasserbeständige Schicht eine Wasserbeständigkeit von mindestens etwa 200 mmH$_2$O aufweist.

7. Einwegwäschestück (20) nach Anspruch 2, wobei der Kanalabschnitt (55) eine zweite wasserbeständige Schicht (93) einschließt, die an die zweite Vliesschicht (94) mit orientierter Faser gefügt ist, und wobei die zweite wasserbeständige Schicht (93) eine Wasserbeständigkeit von mindestens etwa 50 mmH$_2$O aufweist.

8. Einwegwäschestück (20) nach Anspruch 6 oder 7, wobei die wasserbeständige Schicht über eine Wärme/Druck-Bindung oder einen Klebstoff an die Vliesschicht mit orientierter Faser gefügt ist.

9. Einwegwäschestück (20) nach Anspruch 1, wobei die erste Vliesschicht mit orientierter Faser ein Zugfestigkeitsverhältnis von mindestens etwa 15 aufweist.

10. Verbundlaminat (70), das Folgendes umfasst:

eine Vliesschicht mit orientierter Faser mit einer ersten Oberfläche und einer zweiten Oberfläche gegenüber der ersten Oberfläche, wobei die Vliesschicht mit orientierter Faser aus Komponentenfasern mit einer primären Faserrichtung gebildet ist; **dadurch gekennzeichnet, dass**
eine wasserbeständige Schicht an die erste Oberfläche der Vliesschicht mit orientierter Faser gefügt ist, wobei die wasserbeständige Schicht eine Wasserbeständigkeit von mindestens etwa 50 mmH$_2$O aufweist; und

wobei die Vliesschicht mit orientierter Faser ein Faserorientierungsverhältnis wie hierin definiert innerhalb von etwa ±20 Grad von einer primären Faserrichtung von mindestens etwa 65 % aufweist.

### Revendications

1. Vêtement jetable (20) ayant une région antérieure (26), une région postérieure (28) et une région d'entrejambe (30) entre la région antérieure (26) et la région postérieure (28), comprenant :

un châssis (41) fourni dans les régions antérieure, postérieure et d'entrejambe (26, 28, 30), le châssis (41) comprenant une feuille de dessus (24), une feuille de fond (22) associée à la feuille de dessus (24), et une âme absorbante (25) disposée entre la feuille de dessus (24) et la feuille de fond (22), la feuille de fond (22) ayant une surface tournée vers le vêtement ; et
une couverture externe (40) disposée sur la surface tournée vers le vêtement de la feuille de fond, **caractérisé en ce que** le vêtement jetable est une couche à enfiler et dans lequel la couverture externe (40) inclut une première couche de nontissé à fibres orientées ayant un Rapport d'Orientation des Fibres tel que défini ici environ dans les ±20 degrés par rapport à une direction de fibres primaire d'au moins environ 65 %.

2. Vêtement jetable (20) ayant une région antérieure (26), une région postérieure (28) et une région d'entrejambe (30) entre la région antérieure (26) et la région postérieure (28), comprenant :

un châssis (41) fourni dans les régions antérieure, postérieure et d'entrejambe (26, 28, 30), le châssis (41) comprenant une feuille de dessus (24), une feuille de fond (22) associée à la feuille de dessus (24), et une âme absorbante (25) disposée entre la feuille de dessus (24) et la feuille de fond (22) ;
au moins une paire de pans à oreilles étirables (46, 48) s'étendant latéralement vers l'extérieur à partir du châssis (41) dans la région antérieure ou postérieure (26, 28) ; et
un revers formant barrière (54) inclut (a) une partie de canal (55) ayant un bord proximal (59) joint à la feuille de dessus (24) et le bord distal (61) qui est à l'écart de la feuille de dessus (24), et (b) une partie de rabat (57) qui s'étend latéralement vers l'extérieur à partir du bord proximal (59) dans au moins une partie du pan à oreilles (46, 48) ;

**caractérisé en ce que** le vêtement jetable est une couche à enfiler et dans lequel au moins la partie de rabat inclut une deuxième couche de nontissé à fibres orientées ayant un Rapport d'Orientation des Fibres tel que défini ici environ dans les ±20 degrés par rapport à une direction de fibres primaire d'au moins environ 65 %.

**3.** Vêtement jetable (20) selon la revendication 1, comprenant en outre au moins une paire de pans à oreilles étirables (46, 48) s'étendant latéralement vers l'extérieur à partir du châssis (41) dans la région antérieure ou postérieure (26, 28), dans lequel la première couche de nontissé à fibres orientées s'étend latéralement vers l'extérieur à partir du châssis (41) dans la partie du pan à oreilles (46, 48).

**4.** Vêtement jetable (20) selon la revendication 2, dans lequel la deuxième couche de nontissé à fibres orientées s'étend latéralement vers l'extérieur à partir du châssis (41) dans la partie du pan à oreilles (46, 48).

**5.** Vêtement jetable (20) selon la revendication 3 ou 4, dans lequel le pan à oreilles (46, 48) inclut en outre un matériau élastomère (124) joint à la première ou deuxième couche de nontissé à fibres orientées, en formant de ce fait un stratifié élastiquement étirable (70).

**6.** Vêtement jetable (20) selon la revendication 1, dans lequel la feuille de fond (22) inclut en outre une première couche résistant à l'eau jointe à la première couche de nontissé à fibres orientées, et dans lequel la première couche résistant à l'eau a une résistance à l'eau d'au moins environ 200 mmH$_2$O.

**7.** Vêtement jetable (20) selon la revendication 2, dans lequel la partie de canal (55) inclut une deuxième couche résistant à l'eau (93) jointe à la deuxième couche de nontissé à fibres orientées (94), et dans lequel la deuxième couche résistant à l'eau (93) a une résistance à l'eau d'au moins environ 50 mmH$_2$O.

**8.** Vêtement jetable (20) selon la revendication 6 ou 7, dans lequel la couche résistant à l'eau est jointe à la couche de nontissé à fibres orientées à travers une liaison par chaleur/pression ou un adhésif.

**9.** Vêtement jetable (20) selon la revendication 1, dans lequel la première couche de nontissé à fibres orientées a un Rapport de Résistance à la Traction d'au moins environ 15.

**10.** Stratifié composite (70), comprenant :

une couche de nontissé à fibres orientées ayant une première surface et une deuxième surface opposée à la première surface, la couche de nontissé à fibres orientées étant formée à partir de fibres composantes ayant une direction de fibres primaire ; **caractérisé en ce que**
une couche résistant à l'eau est jointe à la première surface de la couche de nontissé à fibres orientées, la couche résistant à l'eau ayant une résistance à l'eau d'au moins environ 50 mmH$_2$O ; et

dans lequel la couche de nontissé à fibres orientées a un Rapport d'Orientation des Fibres tel que défini ici environ dans les ±20 degrés par rapport à une direction de fibres primaire d'au moins environ 65 %.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5782819 A
- US 5171239 A, Igaue
- US 4610681 A, Strohbeen
- WO 9317648 A
- US 4940464 A, Van Gompel
- US 5246433 A, Hasse
- US 5569234 A, Buell
- US 4795454 A
- EP 0843036 A1, Kurihara
- US 5312500 A
- JP H2269859 A
- JP S6025541 B
- US 3860003 A
- US 5151092 A
- US 4610678 A
- US 4673402 A
- US 4888231 A
- US 4834735 A
- US 5190563 A

- US 5234423 A
- US 5147345 A
- EP 0797968 A1, Kurt
- US 4988344 A
- US 4988345 A
- US 3929135 A
- US 4324246 A
- US 4342314 A
- US 4463045 A
- US 5006394 A
- WO 9409736 A, Rollag
- US 4573986 A
- US 3911173 A, Sprague, Jr
- US 4785996 A, Ziecker
- US 4842666 A, Werenicz
- US 4909803 A
- US 4695278 A
- US 4515595 A
- WO 983716 A, Curro